Europäisches Patentamt

European Patent Office

Office européen des brevets

(11) Veröffentlichungsnummer: **0 289 936 B1**

## (12) EUROPÄISCHE PATENTSCHRIFT

(45) Veröffentlichungstag der Patentschrift: **22.09.93**

(21) Anmeldenummer: **88106786.2**

(22) Anmeldetag: **28.04.88**

(51) Int. Cl.5: **C12N 15/12**, C12P 21/02, C12N 1/20, //(C12N1/20, C12R1:465)

(54) **Verfahren zur Herstellung von Fremdproteinen in Streptomyceten.**

(30) Priorität: **05.05.87 DE 3714866**

(43) Veröffentlichungstag der Anmeldung:
**09.11.88 Patentblatt 88/45**

(45) Bekanntmachung des Hinweises auf die
Patenterteilung:
**22.09.93 Patentblatt 93/38**

(84) Benannte Vertragsstaaten:
**AT BE CH DE ES FR GB GR IT LI LU NL SE**

(56) Entgegenhaltungen:
**EP-A- 0 161 629**
**EP-A- 0 281 090**
**DE-A- 3 331 860**

(73) Patentinhaber: **HOECHST AKTIENGESELL-
SCHAFT**

**D-65926 Frankfurt(DE)**

(72) Erfinder: **Koller, Klaus-Peter, Dr.
Carl-Orff-Weg 12
D-6232 Bad Soden am Taunus(DE)**
Erfinder: **Riess, Günther Johannes, Dr.
Loreleistrasse 59
D-6230 Frankfurt am Main 80(DE)**

**Beschreibung**

Aus der europäischen Patentanmeldung mit der Veröffentlichungsnummer (EP-A) 0 161 629 bzw. aus der südafrikanischen Patentschrift 85/3672 ist es bekannt, die für das Signalpeptid (Präpeptid) des α-Amylase-Inhibitors Tendamistat codierende DNA zu nutzen, um ein Polypeptid, insbesondere Tendamistat, aus einer Streptomycetenzelle zu exkretieren. Die entsprechende DNA kann grundsätzlich aus jedem Tendamistat produzierenden Stamm gewonnen werden, bevorzugt wird jedoch eine gemäß Beispiel 3 der deutschen Offenlegunsschrift 3 331 860 erhaltene DNA eingesetzt.

In der Patentanmeldung 88103141.3 (entsprechend der deutschen Patentanmeldung P 37 07 150.5 vom 6. März 1987) wurde bereits ein Verfahren zur Exkretion von Fusionsproteinen aus Streptomyceten vorgeschalgen, das dadurch gekennzeichnet ist, daß man die codierende Sequenz für das gewünschte Protein in das gegebenenfalls modifizierte Tendamistat-Gen einbaut und das rekombinante Gen in einer Streptomycetenzelle exprimiert. Hier wird also das Tendamistat-Strukturgen als "Träger" für ein anderes Gen benutzt, wobei man Fusionsproteine erhält, bei denen die Aminosäuresequenz eines anderen Proteins innerhalb der Tendamistat-Aminosäuresequenz angeordnet ist. Bei der chemischen oder enzymatischen Spaltung dieses Fusionsproteins zur Freisetzung des anderen Proteins erhält man folglich zwei Teilsequenzen des Tendamistats. Die genannte frühere Anmeldung bezieht sich auch auf Tendamistat-Derivate, worunter u. a. auch solche mit deutliche verkürzter Aminosäurekette verstanden werden. Solche Derivate können in reversibler Weise mit den spezifischen Rezeptoren in Form eines kompetitiven Hemmechanismus reagieren.

Es wurde nun gefunden, daß Fremdproteine in Streptomyceten auch dadurch hergestellt werden können, daß man Gene für Fusionsproteine konstruiert, in denen das Strukturgen für das gewünschte Protein an das 3'-Ende (des codierenden Stranges) des gegebenenfalls modifizierten Tendamistat-Gens gekoppelt wird. Die Modifikation des Tendamistat-Gens kann insbesondere in einer Verkürzung am 3'-Ende bestehen.

Die für Tendamistat codierende DNA ist in der EP-A 0 161 629 (dort als DNA-Sequenz C und im Anhang als Tabelle 1) wiedergegeben. In diesem Strukturgen befinden sich mehrere Schnittstellen für Restriktionsenzyme, die zur Modifikation der kodierten Aminosäurefolge genutzt werden können. Geeignet sind die Schnittstellen für BstEII im Bereich der Tripletts 31 und 32, StuI im Bereich der Tripletts 43 und 44 sowie Sau3A im Bereich der Tripletts 52 und 53. Durch Einbau entsprechender Linker können an diesen Stellen eine oder mehrere zusätzliche Aminosäuren eingeschoben werden, DNA-Segmente zwischen diesen Schnittstellen eliminiert werden oder durch Einbau von Stopp-Kodons verkürzte Aminosäuresequenzen kodiert werden. Darüber hinaus können durch ortsspezifische Mutagenese beliebige Aminosäuren eingefügt, ausgetauscht oder eliminiert werden. Man erhält so Proteine, die α-Amylase-Inhibitorwirkung zeigen, oder aber Proteine ohne diese Wirksamkeit, die aber noch mit den entsprechenden Rezeptoren reagieren.

Die Erfindung betrifft weiterhin entsprechende Genstrukturen, diese Genstrukturen enthaltende Vektoren, mit diesen Vektoren transformierte Streptomycetenzellen, die exkretierten Fusionsproteine und deren Verwendung zur Herstellung der Fremdproteine und Tendamistat-Derivate. Bevorzugte Ausgestaltungen der Erfindung werden im folgenden näher erläutert bzw. in den Patentansprüchen definiert.

In den Figuren 1 bis 4 sind einig erfindungsgemäße Plasmidkonstruktionen dargestellt.

Die Fig. 1 zeigt die Herstellung des Hybridplasmids pKK310, das für ein Fusionsprotein codiert, in dem auf einen Teil der Aminosäuresequenz für Tendamistat ein Brückenglied von sieben Aminosäuren und hierauf die Aminosäuresequenz von Affenproinsulin folgt.

Die Fig. 2 zeigt die Überführung des Plasmids pKK310 in das Expressionsplasmid pTF1.

Die Fig. 3 zeigt die Konstruktion des Plasmids pRS10, in dem auf einen Teil des Tendamistatgens der Polylinker aus pUC18 folgt, und dessen Überführung in das Expressionsplasmid pTF10. "mcs" bedeutet die Polylinkerregion (multiple cloning site) von pUC18.

Die Fig. 4 zeigt schließlich die Konstruktion des Plasmids pKK400, das für ein Fusionsprotein codiert, in dem auf die gesamte Aminosäursequenz des Tendamistats ein Brückenglied aus elf Aminosäuren und hierauf die Aminosäuresequenz von Affenproinsulin folgt, und dessen Überführung in das Expressionsplasmid pGF1.

Die Figuren sind nicht maßstäblich gezeichnet.

Die erfindungsgemäß gewonnenen, aus der Zelle exportierten Fusionsproteine bieten den Vorteil, daß sie leicht aus dem Kulturfiltrat isoliert werden können. Die Isolierung kann in an sich bekannter Weise erfolgen, vorteilhaft durch Adsorptions- oder Ionenaustauschchromatographie und/oder Gelfiltration.

Die Freisetzung des gewünschten Fremdproteins (Fusionspartners) durch enzymatische oder chemische Spaltung erfolgt ebenfalls in an sich bekannter Weise.

2

Hierbei richtet sich die Art der Spaltung vor allem nach der Aminosäuresequenz des gewünschten Proteins. In vielen Fällen wird es zweckmäßig sein, in die Spaltstelle zwischen Tendamistat-Sequenz und der Aminosäuresequenz des gewünschten Proteins ein Bindeglied bzw. Brückenglied einzubauen. Wenn das gewünschte Protein beispielsweise kein Methionin enthält, kann das Bindeglied Met bedeuten, worauf eine chemische Spaltung mit Chlor- oder Bromcyan erfolgt. Steht im Bindeglied am Carboxyterminus Cystein oder steht das Bindeglied für Cys, so kann eine enzymatische Cysteinspezifische Spaltung oder eine chemische Spaltung, beispielsweise nach spezifischer S-Cyanylierung, folgen. Steht im Brückenglied am Carboxyterminus Tryptophan oder das Bindeglied für Trp, so kann eine chemische Spaltung mit N-Bromsuccinimid erfolgen.

Gewünschte Proteine, die in ihrer Aminosäuresequenz nicht Asp - Pro enthalten und hinreichend säurestabil sind, können als Fusionsproteine mit diesem Brückenglied in an sich bekannter Weise proteolytisch gespalten werden. Hierdurch erhält man Proteine, die N-terminal Prolin bzw. C-terminal Asparaginsäure enthalten. Auf diese Weise können also auch modifizierte Proteine synthetisiert werden.

Die Asp-Pro-Bindung kann noch säurelabiler gestaltet werden, wenn dieses Brückenglied $(Asp)_n$-Pro bedeutet bzw. Glu-$(Asp)_n$-Pro ist, wobei n 1 bis 3 bedeutet.

Beispiele für enzymatische Spaltungen sind ebenfalls bekannt, wobei auch modifizierte Enzyme mit verbesserter Spezifität eingesetzt werden können (vgl. C.S. Craik et al., Science 228 (1985) 291-197). Ist das gewünschte eukaryotische Peptid Proinsulin so wählt man zweckmäßig eine Peptidsequenz, bei der eine durch Trypsin abspaltbare Aminosäure (Arg. Lys) and die N-terminale Aminosäure (Phe) des Proinsulins gebunden ist, beispielsweise Ala-Ser-Met-Enthält das gewünschte Protein nicht die Aminosäurefolge Ile-Glu-Gly-Art,

so kann das Fusionsprotein mit dem entsprechenden Brückenglied mit Faktor Xa gespalten werden (EP-A 0 025 190 und 0 161 973).

Die Isolierung der Spaltprodukte richtet sich nach den Eigenschaften dieser Proteine. Hinsichtlich der Isolierung von Tendamistat und seinen Derivaten kann auf die in der deutschen Offenlegungsschrift 3 331 860 zitierte Literatur verwiesen werden.

In den folgenden Beispielen wird die Erfindung näher erläutert. Sofern keine anderen Angaben gemacht sind, beziehen sich Protentangaben auf das Gewicht.

Die Beispiele werden durch Figuren gleicher Nummer erläutert. In den Figuren sind die Plasmide und Genfragmente nicht maßstäblich dargestellt; insbesondere in Polylinkersequenzen ist der Maßstab gedehnt.

**Beispiel 1**

Als Ausgangsmaterial dient das Plasmid pKAI650, das in der deutschen Offenlegungsschrift 3 536 182 bzw. in der EP-A 0 218 204 beschrieben ist. Dieses Plasmid ist aus dem in der deutschen Offenlegungsschrift 3 331 860 beschriebenen Plasmid pKAI1 durch Isolieren des 650 bp HincII-SstI-Fragments und Klonieren in das mit diesen Enzymen geöffnete Plasmid pUC19 zugänglich. In diesem Plasmid wird die singuläre HindIII-Schnittstelle (durch Schneiden mit diesem Enzym, Auffüllen der überstehenden Enden und Ligieren) entfernt und so das Plasmid pKAI650a (1) erhalten.

2 μg über CsCl-Gradientenzentrifugation gereinigt DNA von (1) wird 2 Stunden in einem 50 μl-Reaktionsansatz mit StuI nach den Angaben des Herstellers komplett verdaut und das Enzym durch Phenolextraktion entfernt. Die linearisierte DNA wird mit Ethanol gefällt, wieder gelöst und in ein Ligationsgemisch eingebracht, dem als zusätzlicher Reaktionspartner 0,1 μg des chemisch synthetisierten, am 5'-Ende phosphorylierten, doppelsträngigen Oligonukleotids (2)

$$\underline{HindIII}$$

$$5' \quad C \; C \; C \; A \; A \; G \; C \; T \; T \; G \; G \; G \quad 3' \qquad (2)$$
$$3' \quad G \; G \; G \; T \; T \; C \; G \; A \; A \; C \; C \; C \quad 5'$$

zugegeben wird.

Nach Transformation des Ligationsgemisches in E.coli JM 109 werden solche Klone isoliert, die das rekombinante Plasmid pKK3a (3) tragen. Die isolierte Plasmid-DNA ist durch eine Schnittstelle für das Restriktionsenzym HindIII gekennzeichnet, die eine Charakterisierung durch Restriktionsanalyse erlaubt. pKK3a (3) ist um 12 Basenpaare größer als pKAI650a (1) und weist eine Nukleotidsequenz auf, durch die die Aminosäuresequenz wie folgt um 4 Aminosäuren erweitert wird:

```
         42      43                              (44)

         Glu     Gly     Pro     Ser     Leu     Gly     Leu


    5'   GAA     GG C    CCA     AGC     TTG     GG C    CTG     3'
    3'   CTT     CC G    GGT     TCG     AAC     CC G    GAC     5'


                            HindIII
```

Als ein weiteres Ausgangsmaterial dient das Plasmid pYE24 (4). Dieses Plasmid wird dadurch erhalten, daß der Vektor pUC8 mit EcoRI und HindIII geöffnet wird und in dieses linearisierte Plasmid das Affen-Proinsulingen (Tabelle 2; vgl. Wetekam et al., Gene 19 (1982) 179-183) hineinligiert wird.

2 µg des Plasmids pYE24 (4) werden mit den Restriktionsenzymen EcoRI und HindIII umgesetzt, das Affen-Proinsulingen durch Elektroelution isoliert und nach Reinigung und Konzentrierung durch Ethanolfällung mit dem synthetischen DNA-Linker (5)

```
        5' AGC TTG ATG GCG            3'
        3'         AC TAC CGC TTA A  5'              (5)

        (HindIII)           (EcoRI)
```

ligiert. Das Ligationsprodukt (6) wird nunmehr in das mit HindIII geöffnete Plasmid pKK3a (3) eingefügt, wobei das Plasmid pKK31 (7) erhalten wird. Durch diese Konstruktion wird im Anschluß an das Codon für die Aminosäure Gly 43 des Tendamistat-Gens das folgende Brückenglied geschaffen:

```
        43                                      B 1

        Gly   Pro Ser Leu Met Ala Asn Ser Phe
        GGC   CCA AGC TTG ATG GCG AAT TCT TTT
        CCG   GGT TCG AAC TAC CGC TTA AGA AAA
              HindIII          EcoRI
```

"B 1" bezeichnet hier - wie in der Tabelle 2 - den anfang der B-Kette des Affin-Proinsulins.

Im Plasmid (7) befindet sich die Proinsulin-Sequenz innerhalb des Tendamistat-Gens. Zur Überführung dieses Plasmids in ein erfindungsgemäßes Plasmid wird (7) mit SphI und SalI verdaut und das Fragment (8) isoliert. Der Vektor pUC19 wird mit SphI und SalI geöffnet und das linearisierte Plasmid mit dem Fragment (8) ligiert. Das so erhaltene Plasmid pKK310 (9) codiert für ein Fusionsprotein, bei dem auf die verkürzte Tendamistat-Sequenz und den vorstehend dargestellten Linker nur noch die Proinsulinsequenz folgt.

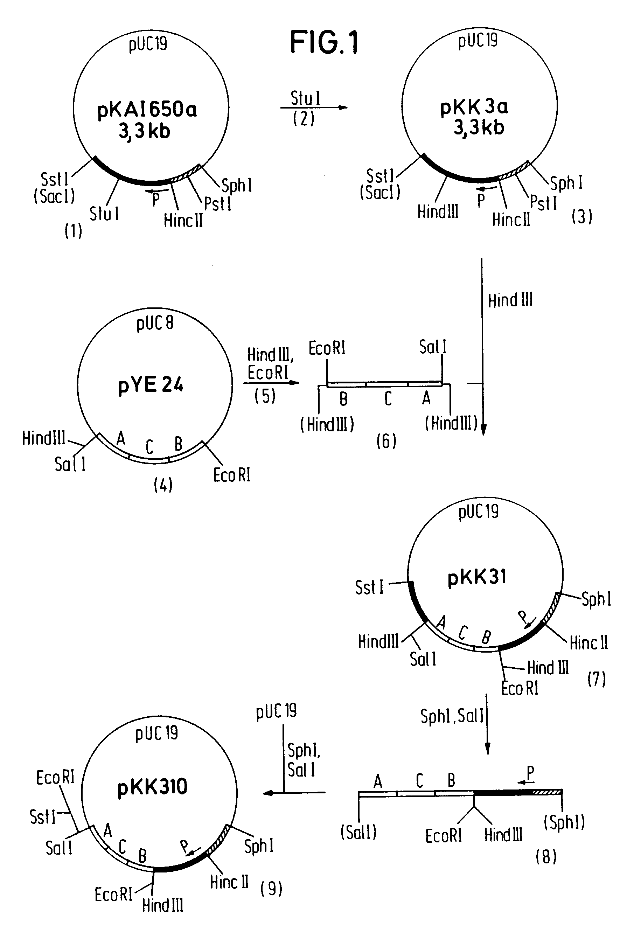

Die gesamte Konstruktion ist in der Figur 1 dargestellt.

**Beispiel 2**

Zur Überführung des Plasmids pKK310 (9) in ein Expressionsplasmid wird (9) mit SstI und SphI umgesetzt und das Fragment (10) isoliert.

Der handelsübliche Expressionsvektor pIJ702 (11) (erhältlich bei der John Innes Foundation, Norwich, England) wird mit SphI und SstI geöffnet und das linearisierte Plasmid (12) mit dem Fragment (10) ligiert. Nach Transformation des Stammes Streptomyces lividans TK 24 (John Innes Foundation) werden die gewünschten Klone durch Selektion auf Resistenz gegenüber Thiostrepton ermittelt. Die Plasmid-DNA aus Thiostrepton-resistenten Klonen wird isoliert und durch Restriktionsanalyse überprüft. Plasmide mit der gewünschten Orientierung des Gens erhalten die Bezeichnung pTF1 (13). Klone, die dieses rekombinante Plasmid enthalten, sekretieren ein Protein mit dem Molekulargewicht 16 kD in das Kulturmedium. Dieses Protein reagiert mit Insulin-Antikörpern im "Immunblot" positiv. (vgl. Beispiel 5).

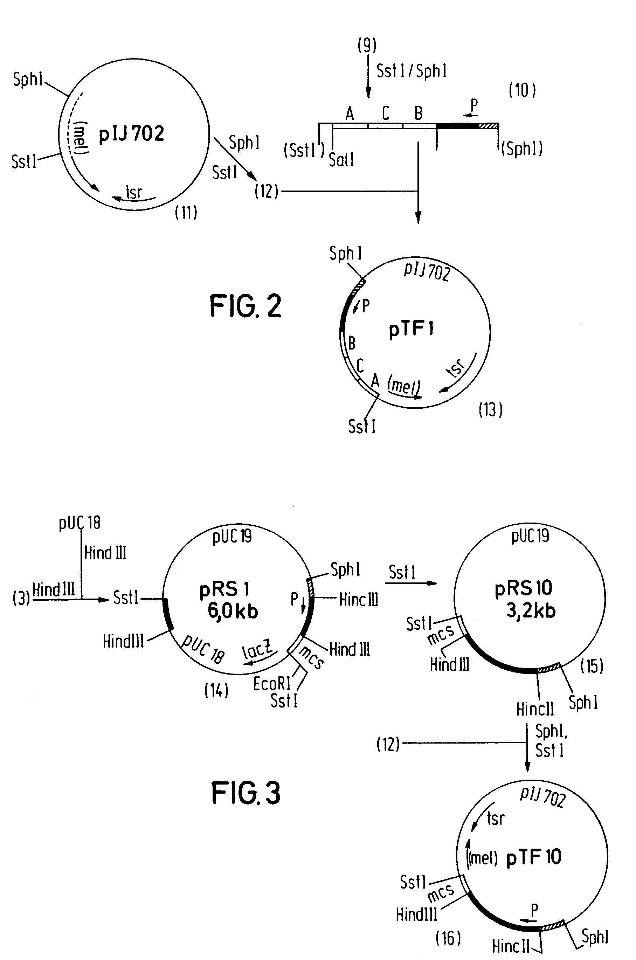

Die Konstruktion von pTF1 (13) ist in der Figur 2 dargestellt.

**Beispiel 3**

Das Plasmid pKK3a (3) einerseits und der Vektor pUC18 andererseits werden jeweils mit HindIII geöffnet und zusammenligiert. Mit dem Ligationsansatz wird der E. coli-Stamm JM 109 transformiert, der die erfolgreiche Klonierung in Gegenwart von Isopropyl-$\beta$-thiogalactopyranosid (IPTG) und 5-Brom-4-chlor-3-indolyl-$\beta$-D-galactopyranosid (X-Gal) durch die Bildung ungefärbter Kolonien anzeigt. Das entstandene rekombinierte Plasmid pRS1 (14) wird in an sich bekannter Weise isoliert. Durch Verdauung von 1 $\mu$g des Plasmids mit dem Restriktionsenzym SstI und nachfolgende Religation wird der pUC18-Anteil bis auf die Polylinkersequenz (mcs) sowie der Rest des Tendamistatgens deletiert. Man erhält das Plasmid pRS10 (15).

Das Plasmid (15) eignet sich durch seinen Polylinkeranteil zur Klonierung beliebiger Strukturgene, wodurch Plasmide erhalten werden, die für die entsprechenden Fusionsproteine mit der verkürzten Tendamistatsequenz codieren.

Verdaut man pRS10 (15) mit SphI und SstI und isoliert das kleinere Fragment, so kann man dieses analog Beispiel 2 in den Expressionsvektor pIJ702 ligieren. Man erhält so den Expressionsvektor pTF10 (16), der ebenfalls auf Grund seines Polylinkeranteils vielseitige Konstruktionen erlaubt.

Die Konstruktion von pTF10 (16) ist in der Figur 3 dargestellt.

**Beispiel 4**

Man öffnet das Plasmid pYE24 (4) mit EcoRI und setzt den Linker

```
5' AAT TCA AGC TTG         3'
3'         GT TCG AAC TTA A  5'
   (EcoRI) Hind III (EcoRI)
```

ein, so erhält man das Plasmid pYE241. Durch Schneiden mit HindIII und Ligieren in mit HindIII geschnittenes pKK3a (3) erhält man analog Beispiel 1 das Plasmid pKK32. Dieses codiert für ein Fusionsprotein, in dem die Tendamistat-Sequenz durch das folgende Brückenglied mit der Proinsulin-Sequenz verknüpft ist:

```
43

Gly Pro Ser Leu Asn Phe Ala Arg
GGC CCA AGC TTG AAT TCT GCA AGA TTT
CCG GGT TCG AAC TTA AGA CGT TCT AAA
```

Analog Beispiel 1 wird pKK32 mit SphI und SstI geschnitten und das ca. 650 bp große Fragment in pUC19, das mit diesen Enzymen geöffnet wurde, umkloniert. Das resultierende Plasmid pKK320 entspricht dem Plasmid pKK310 (9) bis auf das vorstehende Brückenglied (in dem die durch den Linker eingeführte Sequenz durch Fettdruck hervorgehoben ist).

Analog Beispiel 2 wird das SstI-SphI-Fragment mit dem rekombinanten Gen aus pKK320 in pIJ702 umkloniert, wodurch das Expressionsplasmid pTF2 erhalten wird. In S. lividans TK 24 wird ein Fusionsprotein von 16 kd exprimiert und sekretiert, das mit Insulinantikörpern reagiert (vgl. Beispiel 5).

Wegen der Ähnlichkeit der Konstruktion von pTF2 mit pTF1 (13), Figuren 1 und 2, wurde auf eine zeichnerische Darstellung verzichtet.

**Beispiel 5**

pKK310 (9) wird mit EcoRI partiell verdaut, so daß nur eine der beiden EcoRI-Schnittstellen geöffnet wird. Nach Auffüllen der überstehenden Enden mit Hilfe von Klenow-Polymerase wird das Plasmid religiert und durch Restriktionsanalyse das Ergebnis überprüft. Das gewünschte Plasmid, bei dem die am Ende des Proinsulingens stehende EcoRI-Stelle eliminiert wurde, erhält die Bezeichnung pKK310a (17). Dieses enthält also nunmehr eine singuläre Restriktionsstelle für EcoRI in der Linkerregion zwischen dem verkürzten Tendamistatgen und dem Proinsulingen.

Zur Konstruktion eines Plasmids, das für ein Fusionsprotein mit der kompletten Sequenz des Tendamistats codiert, wird in die für Tendamistat codierende DNA-Sequenz (Tabelle 1) im Bereich der Codons für die Aminosäuren 68/69 eine singuläre Schnittstelle für KpnI eingeführt. Dazu wird die isoliert DNA von pKAI650a (1) mit Sst und SphI verdaut und das 650 bp große Fragment in die ebenfalls mit diesen Enzymen doppelt verdaute RF-DNA des Phagen M13mp18 umkloniert und nach bekannten Methoden die Einzelstrang-DNA hergestellt. 1 $\mu$g dieser ssDNA wird zusammen mit 0,1 $\mu$g des mutagenen "Primers"
5′ C GAG GTA CCG GGC GT 3′
in eine "site directed mutagenesis" eingesetzt (M. J. Zoller u. J. Smith, Nucleic Acid Res. 10 (1982) 6487-6500).

Aus den isolierten M13-Klonen mit dem mutierten Gen, das sich durch die zusätzliche KpnI-Schnittstelle selektieren läßt, wird die RF-DNA isoliert und der Basenaustausch (C gegen G an der dritten Position des Codons für Arg[68]) durch Sequenzierung abgesichert. Der Nucleotidaustausch bewirkt also keine Veränderung der Aminosäuresequenz, jedoch die Einführung der gewünschten neuen singulären Schnittstelle in das Tendamistat-Strukturgen.

```
      66   67   68   69   70

     His  Ala  Arg  Tyr  Leu
     CAC  GCC  CGC  TAC  CTC
     GTC  CGG  GCC  ATC  GAG

                    KpnI
```

Die mutierte Sequenz wird nach SstI-SphI-Verdauung aus der M13mp18-RF-DNA in das Plasmid pUC19 umkloniert, wobei das Plasmid pKAI651 (18) erhalten wird.

Zur Kontrolle wird das 650 bp SstI-SphI-Inert aus (18) entsprechend Beispiel 2 in das Plasmid pIJ702 eingebaut, wobei man das Plasmid pAX651 erhält. Nach Transformation dieses Plasmids in Streptomyces lividans TK 24 erhält man die gleichen Expressionsraten für Tendamistat wie für das Plasmid pAX650 mit dem unveränderten Tendamistatgen (deutsche Offenlegungsschrift 3 536 182, Fig. 3).

Zur Herstellung eines erfindungsgemäßen Plasmids für ein Fusionsprotein mit der gesamten Aminosäuresequenz von Tendamistat wird nun das Plasmid pKAI651 (18) mit SphI und KpnI verdaut und das kleine Fragment mit dem Linker (19)

```
      69   70   71   72   73   74

     (Tyr)Leu  Ala  Arg  Cys  Leu  Phe  Asn  Ala  Met  Ala  Thr  Gly          3'
   5'       CTC  GCT  CGC  TGC  CTT  TTC  AAT  GCG  ATG  GCC  ACC  GGG
   3' C  ATG  GAG  CGA  GCG  ACG  GAA  AAG  TTA  CGC  TAC  CGG  TGG  CCC  TTA  A 5'
      (KpnI)                                                         (EcoRI)
                            (19)
```

und dem mit SphI und EcoRI geöffneten Plasmid pKK310a (17) ligiert.

Mit dem Ligationsgemisch wird E. coli JM 109 transformiert, die Plasmid-DNA isoliert und die korrekte Fusion durch DNA-Sequenzierung verifiziert. Das Plasmid mit der korrekten Sequenz erhält die Bezeichnung pKK400 (20).

Der Linker (19) codiert nicht nur für die restlichen Aminosäuren von Tendamistat, sondern auch für den Teil eines "Spacer", der das Tendamistat - und das Proinsulingen - voneinander trennt und insgesamt - mit dem 5'- Ende des Gens nach Tabelle 2 - die Codons für die folgenden 11 Aminosäuren umfaßt:
Phe-Asn-Ala-Met-Ala-Thr-Gly-Asn-Ser-Ala-Arg

Das Fusionsprotein enthält also in diesem "Spacer" unter anderem die Aminosäuren Methionin und Arginin, die eine Spaltung mit Halogencyan bzw. Trypin erlauben.

Aus dem Plasmid pKK400 (20) wird durch Doppelverdauung mit SstI und SphI das Inert von etwa 1090 bp isoliert und die DNA in das mit den gleichen Enzymen geöffnete Plasmid pIJ702 (12) ligiert. Es resultiert das Plasmid pGF1 (21). Das Ligationsgemisch wird in S. lividans TK 24 transformiert und aus Thiostrepton-resistenten Transformanten, die Tendamistat-Aktivität zeigen, die Plasmid-DNA isoliert. Alle positiven Klone enthalten das 1090 bp große SstI-SphI-Insert aus pGF1.

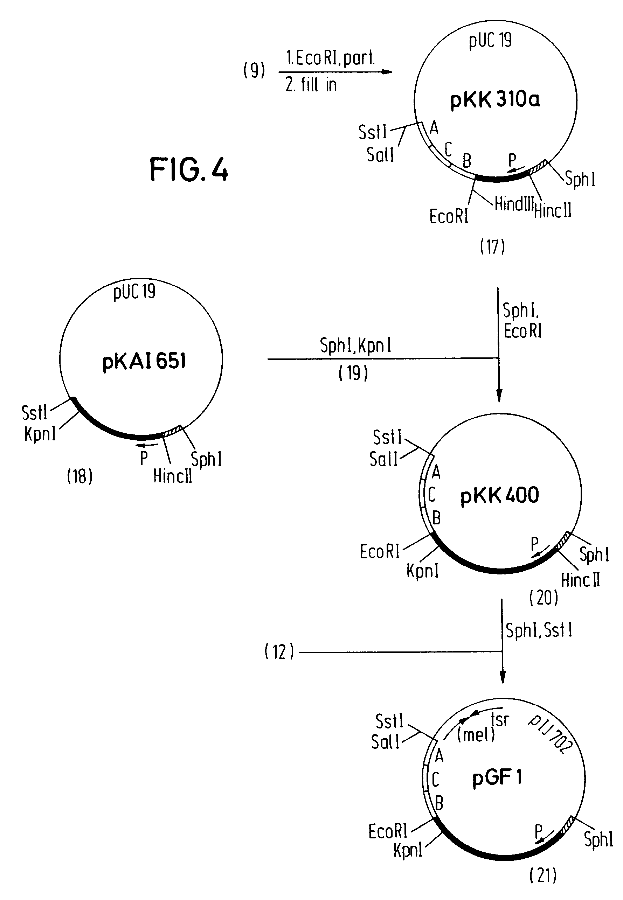

Die Konstruktion von pGF1 (21) ist in der Figure 4 dargestellt.

Die Tendamistat-Aktivität wird nach dem Plattentest ermittelt, der in der EP-A1 0 161 629 in Beispiel 3 sowie in der deutschen Offenlegungsschrift 3 536 182, Beispiel 2, beschrieben ist.

Die Expression des von pGF1 codierten Fusionsproteins kann in bekannter Weise erfolgen. Inkubiert man den transformierten Stamm S. lividans TK 24 vier Tage bei 28°C im Schüttelkolben und trennt das Mycel von der Kulturlösung durch Zentrifugieren ab, so kann man das Fusionsprotein in der klaren Lösung wie folgt nachweisen:

10 bis 100 µl Lösung werden mit 20 bis 200 µl 15%iger Trichloressigsäure versetzt, das ausgefallene Protein durch Zentrifugieren angereichert, gewaschen und in SDS-haltigem Probenpuffer (U. Laemmli, Nature 227 (1970) 680-685) aufgenommen. Nach 2 Minuten Inkubation bei 90°C trennt man elektrophoretisch auf einem 10- bis 17%igen SDS-Polyacrylamidgel auf. Man erhält ein Protein vom Molekulargewicht 19 kD, also im erwarteten Molekulargewichtsbereich für das Fusionsprotein aus Tendamistat und Proinsulin. Das Fusionsprotein reagiert sowohl mit Antikörpern gegen Tendamistat als auch mit Antikörpern gegen Insulin.

**Tabelle 1**

DNA-Sequenz (codierender Strang) und Aminosäuresequenz von Tendamistat

5'-GAC ACG ACC GTC TCC GAG CCC GCA CCC TCC TGC GTG
NH₂-Asp Thr Thr Val Ser Glu Pro Ala Pro Ser Cys Val

ACG CTC TAC CAG AGC TGG CGG TAC TCA CAG GCC GAC
Thr Leu Tyr Gln Ser Trp Arg Tyr Ser Gln Ala Asp

AAC GGC TGT GCC GAG ACG GTG ACC GTG AAG GTC GTC
Asn Gly Cys Ala Glu Thr Val Thr Val Lys Val Val

TAC GAG GAC GAC ACC GAA GGC CTG TGC TAC GCC GTC
Tyr Glu Asp Asp Thr Glu Gly Leu Cys Tyr Ala Val

GCA CCG GGC CAG ATC ACC ACC GTC GGC GAC GGC TAC
Ala Pro Gly Gln Ile Thr Thr Val Gly Asp Gly Tyr

ATC GGC TCG CAC GGC CAC GCG CGC TAC CTG GCT CGC
Ile Gly Ser His Gly His Ala Arg Tyr Leu Ala Arg

TGC CTT TAG-3'
Cys Leu Stp

**Tabelle 2**

```
                                      5' AAT TCT GCA AGA
                                      3'     GA CGT TCT
                                      (Asn)Ser Ala Arg
                                         (EcoRI)


B 1
TTT GTG AAC CAG CAC CTG TGC GGC TCC CAC CTA GTG GAA GCT CTC
AAA CAC TTG GTC GTG GAC ACG CCG AGG GTG GAT CAC CTT CGA GAG
Phe Val Asn Gln His Leu Cys Gly Ser His Leu Val Glu Ala Leu



TAC CTG GTG TGC GGG GAG CGA GGC TTC TTC TAC ACA CCC AAG ACC
ATG GAC CAC ACG CCC CTC GCT CCG AAG AAG ATG TGT GGG TTC TGG
Tyr Leu Val Cys Gly Glu Arg Gly Phe Phe Tyr Thr Pro Lys Thr


C 1
CGC CGG GAG GCA GAG GAC CCT CAG GTG GGG CAG GTG GAG CTG GGC
GCG GCC CTC CGT CTC CTG GGA GTC CAC CCC GTC CAC CTC GAC CCG
Arg Arg Glu Ala Glu Asp Pro Gln Val Gly Gln Val Glu Leu Gly



GGG GGC CCT GGC GCA GGC AGC CTG CAG CCC TTG GCG CTG GAG GGG
CCC CCG GGA CCG CGT CCG TCG GAC GTC GGG AAC CGC GAC CTC CCC
Gly Gly Pro Gly Ala Gly Ser Leu Gln Pro Leu Ala Leu Glu Gly


                        A 1
TCC CTG CAG AAG CGC GGC ATC GTG GAG CAG TGC TGC ACC AGC ATC
AGG GAC GTC TTC GCG CCG TAG CAC CTC GTC ACG ACG TGG TCG TAG
Ser Leu Gln Lys Arg Gly Ile Val Glu Gln Cys Cys Thr Ser Ile



TGC TCC CTC TAC CAG CTG GAG AAC TAC TGC AAC TAA TAG TCG ACC
ACG AGG GAG ATG GTC GAC CTC TTG ATG ACG TTG ATT ATC AGC TGG
Cys Ser Leu Tyr Gln Leu Glu Asn Tyr Cys Asn
                                             SalI


TGC AGC CA            3'
ACG TCG GTT CGA       5'
PstI      (HindIII)
```

**B 1, C 1 und A 1 bezeichnen den Beginn der B-, C- und A-Kette des Affenproinsulins**

**Patentansprüche**

**Patentansprüche für folgende Vertragsstaaten : AT, BE, CH, DE, FR, GB, GR, LI, LU, NL, SE**

1. Verfahren zur Herstellung von Fusionsproteinen, dadurch gekennzeichnet, daß man das Strukturgen für das gewünschte Protein an das 3'-Ende (des codierenden Stranges) des gegebenenfalls modifizierten Tendamistat-Gens koppelt, diese Genstruktur in einer Streptomyceten-Wirtszelle zur Expression bringt und das sekretierte Fusionsprotein aus dem Überstand isoliert.

2. Verfahren nach Anspruch 1, dadurch gekennzeichnet, daß das Tendamistat-Gen am 3'-Ende verkürzt ist.

3. Genstruktur, enthaltend das gegebenenfalls modifizierte Tendamistat-Gen, an dessen 3'-Ende ein Strukturgen für ein anderes Protein gekoppelt ist.

4. Genstruktur nach Anspruch 3, dadurch gekennzeichnet, daß das Tendamistatgen am 3'-Ende verkürzt ist.

5. Vektor, enthaltend eine Genstruktur nach Anspruch 3 oder 4.

6. Streptomycetenzelle, enthaltend einen Vektor nach Anspruch 5.

7. Fusionsprotein, dadurch gekennzeichnet, daß es nur einen N-ständigen Anteil von gegebenenfalls modifiziertem Tendamistat hat.

8. Verwendung des Fusionsproteins nach Anspruch 7 bzw. des nach Anspruch 1 oder 2 erhältlichen Fusionsproteins zur Herstellung des gegebenenfalls modifizierten Tendamistats und/oder des Fusionspartners.

**Patentansprüche für folgenden Vertragsstaat : ES**

1. Verfahren zur Herstellung von Fusionsproteinen, dadurch gekennzeichnet, daß man das Strukturgen für das gewünschte Protein C-terminal an das gegebenenfalls modifizierte Tendamistatgen koppelt, diese Genstruktur in einer Streptomyceten-Wirtszelle zur Expression bringt und das sekretierte Fusionsprotein aus dem Überstand isoliert.

2. Verfahren nach Anspruch 1, dadurch gekennzeichnet, daß das Tendamistat-Gen C-terminal verkürzt ist.

3. Verfahren zur Herstellung der in Anspruch 1 eingesetzten Genstruktur, dadurch gekennzeichnet, daß man C-terminal an das gegebenenfalls modifizierte Tendamistat-Gen ein Strukturgen für ein anderes Protein koppelt.

4. Verfahren nach Anspruch 3, dadurch gekennzeichnet, daß das Tendaminstatgen C-terminal verkürzt ist.

5. Verfahren zur Herstellung eines Proteins aus genetisch codierbaren Aminosäuren, dadurch gekennzeichnet, daß man aus einem nach Anspruch 1 oder 2 erhaltenen Fusionsprotein den Tendamistat-Anteil abspaltet.

**Claims**
**Claims for the following Contracting States : AT, BE, CH, DE, FR, GB, GR, LI, LU, NL, SE**

1. A process for the preparation of fusion proteins, which comprises coupling of the structural gene for the desired protein onto the 3' end (of the coding strand) of the tendamistat gene, which is modified where appropriate, bringing about the expression of this gene structure in a Streptomyces host cell, and isolating the secreted fusion protein from the supernatant.

2. The process as claimed in claim 1, wherein the tendamistat gene is truncated at the 3' end.

3. A gene structure containing the tendamistat gene, which is modified where appropriate, and onto the 3' end of which a structural gene for another protein has been coupled.

4. A gene structure as claimed in claim 3, wherein the tendamistat gene is truncated at the 3' end.

5. A vector containing a gene structure as claimed in claim 3 or 4.

10

6. A Streptomyces cell containing a vector as claimed in claim 5.

7. A fusion protein which has only one N-terminal portion of tendamistat, which is modified where appropriate.

8. The use of the fusion protein as claimed in claim 7, or of the fusion protein obtainable as claimed in claim 1 or 2, for the preparation of tendamistat, which is modified where appropriate, and/or of the fusion partner.

**Claims for the following Contracting State : ES**

1. A process for the preparation of fusion proteins, which comprises C-terminal coupling of the structural gene for the desired protein onto the tendamistat gene, which is modified where appropriate, bringing about the expression of this gene structure in a Streptomyces host cell, and isolating the secreted fusion protein from the supernatant.

2. The process as claimed in claim 1, wherein there is C-terminal truncation of the tendamistat gene.

3. A process for the preparation of the gene structure employed in claim 1, which comprises C-terminal coupling onto the tendamistat gene, which is modified where appropriate, of a structural gene for another protein.

4. The process as claimed in claim 3, wherein there is C-terminal truncation of the tendamistat gene.

5. A process for the preparation of a protein from genetically encodable amino acids, which comprises cleaving the tendamistat portion off a fusion protein obtained as claimed in claim 1 or 2.

**Revendications**
**Revendications pour les Etats contractants suivants : AT, BE, CH, DE, FR, GB, GR, LI, LU, NL, SE**

1. Procédé pour la production de protéines de fusion, caractérisé en ce que l'on soude le gène structural, codant pour la protéine recherchée, à l'extrémité 3' (du brin codant) du gène du tendamistat éventuellement modifié, on exprime cette structure génique dans une cellule hôte de Streptomyces et on isole à partir du surnageant la protéine de fusion sécrétée.

2. Procédé selon la revendication 1, caractérisé en ce que le gène du tendamistat est raccourci à l'extrémité 3'.

3. Structure génique contenant le gène du tendamistat éventuellement modifié, à l'extrémité 3' duquel est soudé une structure génique codant pour une autre protéine.

4. Structure génique selon la revendication 3, caractérisée en ce que le gène du tendamistat est raccourci à l'extrémité 3'.

5. Vecteur contenant un gène structural selon la revendication 3 ou 4.

6. Cellule de Streptomyces contenant un vecteur selon la revendication 5.

7. Protéine de fusion, caractérisée en ce qu'elle ne comporte qu'une fraction en N de tendamistat éventuellement modifié.

8. Utilisation de la protéine de fusion selon la revendication 7 ou de la protéine de fusion pouvant être obtenue selon la revendication 1 ou 2, pour la production du tendamistat éventuellement modifié et/ou du partenaire de fusion.

**EP 0 289 936 B1**

**Revendications pour l'Etat contractant suivant : ES**

1. Procédé pour la production de protéines de fusion, caractérisé en ce que l'on soude le gène structural, codant pour la protéine recherchée, à l'extrémité 3' du gène du tendamistat éventuellement modifié, on exprime cette structure génique dans une cellule hôte de Streptomyces et on isole à partir du surnageant la protéine de fusion sécrétée.

2. Procédé selon la revendication 1, caractérisé on ce que le gène du tendamistat est raccourci à l'extrémité 3'.

3. Procédé pour la production de la structure génique utilisée dans la revendication 1, caractérisé en ce que l'on soude une structure génique, codant pour une autre protéine, à l'extrémité 3' du gène du tendamistat éventuellement modifié.

4. Structure génique selon la revendication 3, caractérisée en ce que le gène du tendamistat est raccourci à l'extrémité 3'.

5. Procédé pour la production d'une protéine constituée d'aminoacides génétiquement codables, caractérisé en ce que l'on sépare le fragment tendamistat à partir d'une protéine de fusion obtenue selon la revendication 1 ou 2.

12

FIG.1

FIG. 2

FIG. 3

14

# FIG. 4